Europäisches Patentamt

(19) European Patent Office

Office européen des brevets

(11) Numéro de publication: **0 201 390**
**B1**

(12) **FASCICULE DE BREVET EUROPEEN**

(45) Date de publication du fascicule du brevet:
04.07.90

(51) Int. Cl.⁵: **A61K 31/715, A23L 1/03**

(21) Numéro de dépôt: **86400831.3**

(22) Date de dépôt: **17.04.86**

(54) **Perfectionnements apportés à la préparation des inhibiteurs de l'adhésion cellulaire, antiadhésines ainsi obtenues et médicaments et aliments les contenant.**

(30) Priorité: **23.04.85 FR 8506124**

(43) Date de publication de la demande:
**17.12.86 Bulletin 86/46**

(45) Mention de la délivrance du brevet:
**04.07.90 Bulletin 90/27**

(84) Etats contractants désignés:
**AT BE CH DE FR GB IT LI LU NL SE**

(56) Documents cités:
**BIOLOGICAL ABSTRACTS,
vol. 70, no. 5, 1980, no. 31311, Philadelphia, Pa., US; A.-K. JARVINEN et al.: "Urinary oligosaccharides inhibit adhesion of Escherichia coli onto canine urinary tract epithelium", & INVEST. UROL 17(6): 433-445, 1980
A.K. Järvinen, Invest. Urol. 17(6), 443-445 (1980)**

(73) Titulaire: **INSTITUT BIO-LIMOUSIN, 123 rue Albert Thomas, F-87060 Limoges Cedex(FR)**

(72) Inventeur: **Julien, Raymond, 65-67 Avenue Baudin, F-87000 Limoges(FR)**
Inventeur: **Mouricout, Michèle, 14 rue Vardelle, F-87100 Limoges(FR)**

(74) Mandataire: **Orès, Bernard et al, Cabinet ORES 6, Avenue de Messine, F-75008 Paris(FR)**

## Description

La présente invention est relative à des perfectionnements apportés à la préparation des inhibiteurs de l'adhésion cellulaire, aux antiadhésines ainsi obtenues et aux médicaments et aliments les contenant.

Il est actuellement universellement reconnu qu'une très grande proportion de microorganismes et notamment de microorganismes pathogènes, a la propriété de se fixer sur la surface du milieu environnant (corps solide quelconque, cellules humaines, animales, végétales). Les microorganismes infectieux adhèrent aux cellules cibles grâce à des structures moléculaires organisées en capsides, membranes externes et/ou pili, fimbriae, flagelles ou autres, appelées d'une façon générale adhésines.

Dans de nombreux cas, le mécanisme d'infection nécessite d'une manière absolue une adhésion préalable du microorganisme sur le milieu environnant ou directement sur un des récepteurs membranaires parfois spécifiques, préalable indispensable à toute colonisation bactérienne. La nature de ces structures microbiennes ou adhésines et celle des récepteurs responsables des phénomènes d'adhésion, n'est pas encore complètement élucidée, mais de nombreux travaux publiés par différents auteurs, cernent de mieux en mieux ce phénomène (Cf. notamment, les travaux de G. Chabanon dans "Revue de l'Inst. Pasteur de Lyon", 1984, t. 18, p. 85-105, de G.W. Jones dans "Microbial Interactions" Série B, Vol. 3, p. 141-176 (1977) ; de A. Gunnarsson et collab. dans "Infection and Immunity", Juillet 1984, p. 41-46, etc...). Il en résulte que les adhésines agissent grâce à des interactions parfois spécifiques qui s'établissent entre certains de leurs sites ou de leurs motifs structuraux et de groupements chimiques présents dans l'environnement proche de la membrane externe de la cellule ou structure cible et/ou à sa surface elle-même.

Parmi les différents moyens actuellement employés destinés à lutter contre les agents infectieux, les deux suivants sont particulièrement utilisés:

– la vaccination (action préventive)
– l'antibiothérapie (action curative).

Toutefois, depuis un certain temps (Cf. Sharon et coll. dans "Adhesion and Microorganism Pathogenicity" Ciba Foundation Symposium 80, p. 119-135; OLD D. C. dans J. gen. Microbiol. 1972, 71, p. 149-157; et JONES G. W. dans la publication précitée) on a préconisé une méthode de lutte anti-infectieuse consistant à inhiber la propriété de l'adhésion du microorganisme. Cette inhibition par compétition met en œuvre des sucres simples de structure bien définie comme les monosaccharides. D'autres auteurs (JÄRVINEN et SANDHOLM, Investigative Urology, Vol. 17, No 16 rapportent l'effet inhibiteur de petits oligosaccharides de structure chimique non définie, et de poids moléculaire inférieur à 1000, extraits de l'urine par filtration. Les résultats obtenus, tout en étant encourageants, n'ont toutefois pas permis de résoudre le problème de manière satisfaisante.

La Demanderesse, en étudiant à fond le phénomène d'adhérence bactérienne, notamment chez les animaux, s'est fixée pour but de perfectionner la méthode d'inhibition par compétition et de développer des agents thérapeutiques nouveaux, des anti-adhésines, capables d'empêcher l'adhérence des bactéries pathogènes: elle s'est fixée pour but de pourvoir à un procédé de préparation des anti-adhésines dont l'action sera à la fois curative et préventive:

– curative, lorsque l'agent infectieux sera déjà présent à proximité ou au contact de la structure ou de la cellule cible, en provoquant son détachement,
– préventive lorsque le risque d'une infection sera élevé et que l'administration de l'anti-adhésine empêchera l'agent pathogène d'atteindre la cible.

La Demanderesse a constaté que les interactions chimiques impliquées dans le phénomène de l'adhésion cellulaire appartiennent à de nombreux modèles. Certains sont spécifiques et sont basés sur un phénomène de reconnaissance moléculaire entre une macromolécule, et général protéique (lectine ou autre), et un récepteur membranaire ; d'autres sont non spécifiques et mettent en jeu des phénomènes de charge de type hydrophobe, électrostatique ou ionique ou de liaisons hydrogène. En général, ces derniers ne sont pas suffisants pour assurer une adhésion durable, mais ils participent souvent à la stabilisation des interactions spécifiques. Dans ce dernier cas, les motifs moléculaires mis en jeu au niveau des récepteurs,sont en général de nature glucidique.

La présente invention a précisément pour but de pourvoir à un procédé de préparation de substances qui peuvent être considérées du point de vue chimique, comme des structures mimétiques des récepteurs engagés dans le phénomène de reconnaissance.

La présente invention est relative à un procédé de préparation d'inhibiteurs de l'adhésion cellulaire ou anti-adhésines, lequel procédé est caractérisé en ce que l'on lyse enzymatiquement et/ou chimiquement les glycoprotéines et/ou les glycopeptides, les structures lipidiques et/ou les glycolipides contenus ou provenant de différents substrats organiques, liquides ou solides, en ce que l'on recueille les structures résistantes à la lyse, de nature partiellement ou totalement osidique, de masse toujours supérieure à 1000, et en ce que l'on procède à leur stérilisation.

Les antiadhésines ainsi obtenues qui peuvent être, si on le désire, greffées sur différents supports, s'opposent à l'adhérence de nombreux microorganismes et constituent un médicament de choix pour combattre ou prévenir l'infection.

Suivant un mode de réalisation avantageux du procédé objet de la présente invention, les glycoprotéines et/ou les glycolipides sont traités sans les avoir extraits du substrat organique.

Suivant un autre mode de réalisation avantageux du procédé objet de la présente invention, on procède à l'hydrolyse des glycoprotéines et/ou glycopeptides et/ou glycolipides après leur extraction du milieu biologique les contenant.

Suivant une modalité de ce mode de réalisation, les glycoprotéines sont extraites par :

a) traitement en milieu organique par l'éthanol en présence du caprylate de Na suivi de,
b) traitement thermique (60 à 70°C) et séparation des glycoprotéines précipitées, et enfin
c) protéolyse suivie éventuellement par une hydrazinolyse.

Suivant une autre modalité de ce mode de réalisation, les glycolipides sont extraits par action d'un mélange de solvants puis soumis à l'hydrolyse.

Les solvants utilisés pour l'extraction des glycolipides peuvent être constitués par un mélange binaire chloroforme-méthanol ou le mélange ternaire chloroforme-éthanol-acétone.

Conformément à l'invention, l'hydrolyse est effectuée :

1. dans le cas des glycoprotéines, à l'aide d'une protéase et de manière préférentielle à l'aide de la thermolysine en présence d'ions calcium à un pH compris entre 5 et 7 et à une température inférieure ou égale à 80°C suivie d'une hydrazinolyse ou traitement chimique analogue permettant de libérer les antiadhésines constitutives.

2. dans le cas de glycolipides par une hydrolyse alcaline douce, suivie d'une acétolyse ou traitement analogue afin d'isoler les structures oligosaccharidiques.

Selon un mode de réalisation avantageux du procédé objet de la présente invention, on procède à la purification des espèces oligosaccharidiques et notamment, on procède après l'hydrolyse enzymatique à l'inactivation de l'activité enzymatique ou à une neutralisation dans le cas d'une lyse chimique, puis à une filtration moléculaire permettant de recueillir les espèces de masse supérieure à 1000, puis après la clarification du filtrat on stérilise sur membrane filtrante.

Suivant un mode de réalisation particulièrement avantageux, on procède avant la stérilisation à la concentration de la solution d'antiadhésines. Cette concentration peut être effectuée par des moyens classiques, tels qu'ultrafiltration, la précipitation, la chromatographie d'exclusion moléculaire, par les résines échangeuses d'ions ou la chromatographie d'affinité.

La présente invention a également pour objet des antiadhésines obtenues à l'aide du procédé conforme à l'invention, lesquelles antiadhésines sont caractérisées en ce qu'elles sont de nature partiellement ou totalement oligosaccharidique, comprenant au minimum 3 oses simples ou substitués, l'un des oses étant obligatoirement le galactose ou le glucose, la masse de l'antiadhésine étant toujours supérieure à 1000.

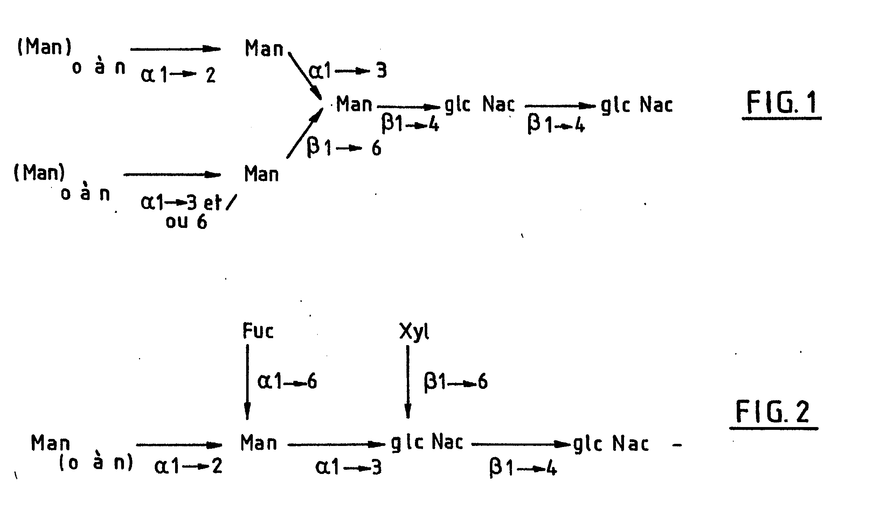

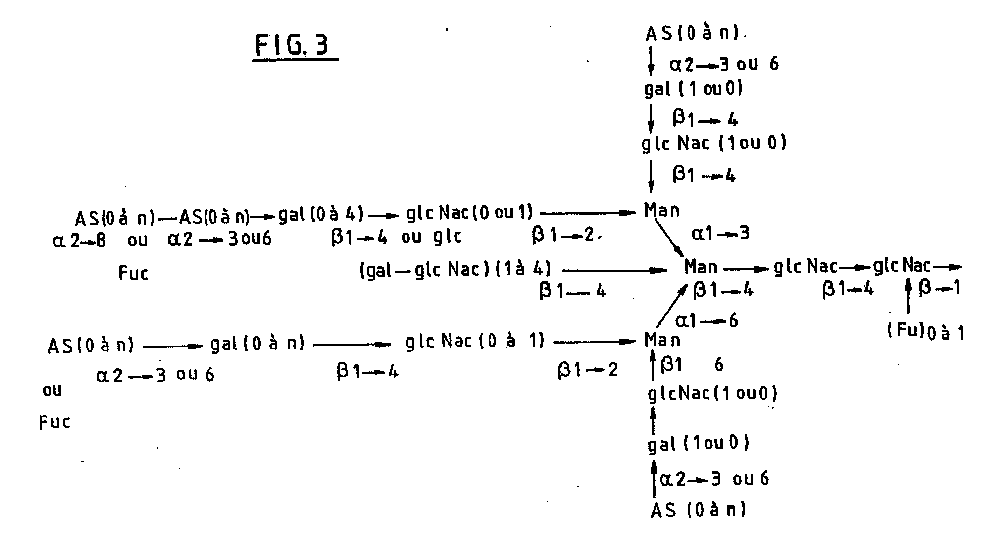

Ces molécules constituent des excellents inhibiteurs d'adhérence et peuvent être administrées sous formes galéniques diverses et peuvent être également mélangées à des aliments. Ces molécules peuvent être de configurations très variées. Elles présentent dans leurs structures des noyaux, ou core ou squelette et des séquences périphériques, terminales et/ou antennaires. Elles possèdent au moins gal ou glc simples ou substitués. Les figures 1 à 3 représentent quelques-unes de ces structures à titre d'exemple non limitatif. Ainsi :

- la figure 1 représente une structure de glycannes de type oligomannosidique d'origine animale,
- la figure 2 représente une structure de glycannes de type oligomannosidique d'origine végétale et

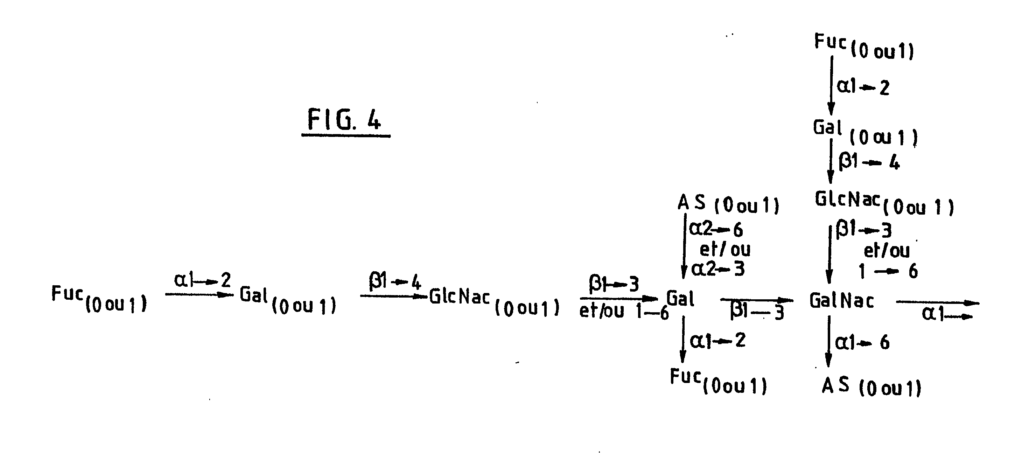

- les figures 3 et 4 représentent le type de structure de n glycannes contenant des unités N-acétyl lactosaminiques répétées ou non substituées ou non,

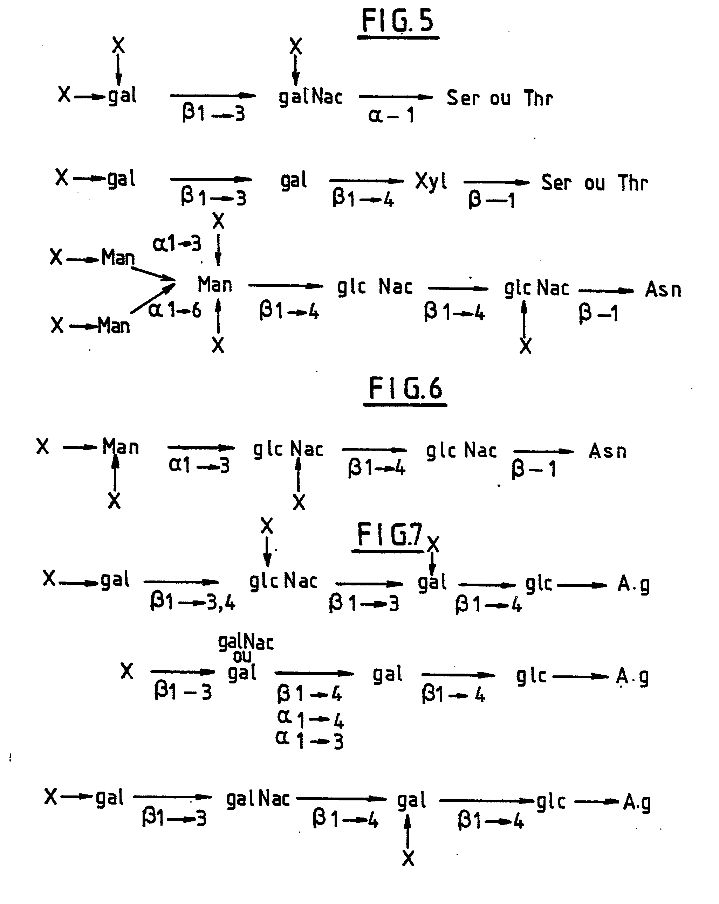

- les figures 5 à 7 représentent quelques exemples de séquences internes des noyaux greffés à des supports :
. glycopeptide d'origine animale sur des supports protéiques ou peptidiques (fig.5),
. glycopeptide d'origine végétale sur des supports protéiques ou peptidiques (fig.6),
. antiadhésine greffée sur des supports lipidiques (fig.7).

Les abréviations qui y figurent sont les suivantes :

Man =mannose
glc =glucose
gal =galactose
X = résidu glucidique
Ser = sérine
Asn = asparagine

glc Nac = N acétyl-glucosamine
gal Nac = N acétyl-galactosamine
Fuc = fucose
AS = acides sialiques
Thr = thréonine
A.g. = acide gras
xyl : xylose

Pratiquement tous les substrats organiques conviennent pour préparer les antiadhésines conformes à la présente invention.

La Demanderesse a ainsi utilisé :
- parmi les liquides biologiques : le plasma sanguin, le plasma sanguin foetal, les erythrocytes, l'urine, la lymphe, le liquide céphalorachidien, le liquide amniotique, le liquide synovial ;
- parmi les secrétions de différentes espèces humaine et animale : le lait, le colostrum, la mucine gastrique, la salive, le mucus ;
- les secrétions végétales ;
- parmi les sources procaryotes et eucaryotes, extraits cellulaires totaux de cellules normales ou transformées par infection : les virus, les phages, les bactéries, les champignons, les levures, les algues, les mousses, les lichens, les fougères, les cellules animales supérieures, les cellules végétales supérieures ;
- parmi les différents extraits : les extraits cellulaires embryonnaires, glandulaires, tissulaires, la cervelle et les tissus nerveux, les yeux, le thymus, la thyroïde, le pancréas, les poumons, les reins, le foie, la rate, les intestins, les os, les muscles, le placenta.

L'invention sera mieux comprise à l'aide de compléments de description qui va suivre, qui se réfère à des exemples de préparation de produits conformes à la présente invention ainsi qu'à des compte-rendus pharmacologiques et cliniques relatifs à l'activité de médicaments conformes à la présente invention.

Les exemples de préparation qui suivent, ainsi que les tests de mise en évidence de l'activité inhibitrice des antiadhésines sont donnés uniquement à titre d'illustration de l'objet de l'invention, dont ils ne constituent en aucune manière, une limitation.

## EXEMPLES DE PREPARATION

### A. ANTIADHESINES DE PLASMA SANGUIN DE DIFFERENTES ESPECES

#### Exemple 1

Le sang recueilli sous anticoagulant est centrifugé et séparé en deux phases : le plasma et le cruor (suspension d'érythrocytes). Le plasma est hydrolysé par une protéase bactérienne thermostable telle que la thermolysine qui se trouve sous forme d'un lyophilisat contenant 25 % de sels de tampon acétate de Na et de Ca (Cf Brevet français 83 21033).

Après inactivation de l'activité enzymatique par traitement thermique (à 75°C au moins) ou par précipitation chimique, les antiadhésines de masse supérieure à 1000 sont recueilles, puis clarifiées, délipidées sur filtre presse par des adjuvants de filtration et stérilisées sur membrane écran de 0,22 μm. Les antiadhésines sont ou peuvent être obtenues par des traitements chimiques particuliers, hydrazinolyse et/ou par des endoglycosidases spécifiques. Le rendement en antiadhésines est d'environ 5% par rapport à la matière protéique de départ.

#### Exemple 2

Le plasma est traité par l'éthanol (8 %) et le caprylate de sodium ($4.10^{-3}$M). Après un traitement thermique pendant 15 min à 68°C, les glycoprotéines précipitées sont séparées de l'albumine par filtration sur filtre presse en présence d'adjuvants.

Le "gateau" de glycoprotéines lavé constitue le substrat de protéolyse. Le rendement en antiadhésines est d'environ 10 %.

#### Exemple 3

Les antiadhésines obtenues par hydrolyse selon les exemples 1 et 2, sont concentrées par des méthodes physico-chimiques : ultrafiltration, précipitations sélectives, chromatographies d'exclusion moléculaire, d'échanges d'ions, d'affinité. Cette dernière technique est utilisée pour séparer les espèces individuelles spécifiques.

B.-ANTIADHESINES DE MEMBRANES D'ERYTHROCYTES DE DIFFERENTES ESPECES.

Exemple 4

Les globules rouges obtenus par centrifugation d'un sang récolté sous anticoagulant sont lavés et les membranes cellulaires sont préparées suivant un procédé modifié de celui présenté par A. FAURE, J. DEBRIEUX et M. CARON (Red. Cell. Ghosts : a tool for purification of lectins in lectins, 1983, vol. 3, pp. 661-665, ed. Walter de Gruyter, Berlin). Le rendement en membranes lyophilisées est environ de 4 g par litre de sang.

Exemple 5

Les membranes d'érythrocyte obtenues selon l'exemple 4 sont soumises à un traitement protéolytique similaire à celui décrit dans l'exemple 1, ce qui permet d'obtenir les antiadhésines portées par des structures de nature protéique.

Exemple 6

Les membranes d'érythrocyte obtenues selon l'exempla 4 sont soumises à un procédé d'extraction des fractions glycolipidiques. Les fractions lipidiques sont obtenues par action d'un mélange de chloroforme et de méthanol en proportion 2:1 et une deuxième extraction par le même mélange en proportion 1:9 ou par un mélange ternaire chloroforme - éthanolacétone dans les proportions 2:1:30. Les extraits obtenus sont soumis à une hydrolyse alcaline douce suivie d'une nouvelle partition et fractionnement par chromatographie sur gel de silice. L'élution est faite par des mélanges méthanol (x vol) chloroforme (y vol) tel que x/y augmente de 1:9 à 20:1.

Après une hydrolyse chimique ou un traitement d'acétolyse, des méthodes chromatographiques suivant un procédé modifié présenté par A.GUNNARSSON & Al. dans "Infection and Immunity" Juillet 1984, p. 41-46 permettent d'isoler les structures oligosaccharidiques individuelles de masse moléculaire toujours supérieure à 1000. Le rendement est d'environ 1%. Toutes les fractions obtenues possèdent au moins Gal et Glc simples ou substitués.

C. - ANTIADHESINES ISSUES DE LIQUIDES BIOLOGIQUES AUTRES QUE LE SANG

Exemple 7

Après concentration, les matières premières subissent un traitement protéolytique selon l'exemple 1 ou un procédé d'extraction des glycolipides selon l'exemple 6.

Le rendement est de l'ordre de 0,5 - 1 %.

D. - ANTIADHESINES OBTENUES A PARTIR DE SECRETIONS MAMMAIRES DE DIFFERENTES ESPECES ANIMALES ET/OU HUMAINE

Exemple 8

Le colostrum ou le lait subit un traitement protéolytique et une purification selon l'exemple 1 et/ou un traitement extractif des structures lipidiques selon l'exemple 6.

E. - ANTIADHESINES OBTENUES A PARTIR DE MUCUS D'ESPECES ANIMALES, EXEMPLE DU MUCUS INTESTINAL

Exemple 9

Le mucus du duodénum, jejunum et iléon est recueilli par grattage. Le matériel biologique mis en suspension est débarrassé des contaminants et débris cellulaires par centrifugation à 20 000 g. Après dialyse, la solution de glycoprotéines mucosales , filtrée stérilement , constitue le substrat de protéolyse selon l'exemple 1.

F. - ANTIADHESINES ISSUES DE GLYCOPEPTIDES ET DE GLYCOLIPIDES INTESTINAUX

Exemple 10

Les villosités et les cellules épithéliales sont récupérées par grattage du duodénum, jéjunum et iléon de l'animal sacrifié et sont lavées par centrifugation. Après homogénéisation au Potter ou hydrolyse partielle à la trypsine (15 minutes à 37°C), les lipides sont extraits par un mélange chloroforme - méthanol - eau (4:8:3 v/v). Deux fractions sont ainsi obtenues, les lipides et glycolipides et les cellules délipidées.

Les extraits glycolipidiques sont traités selon le procédé de l'exemple 6. Les cellules épithéliales délipidées sont hydrolysées selon la méthode décrite dans l'exemple 1.

## G. -ANTIADHESINES ISSUES DE SECRETIONS VEGETALES

### Exemple 11

Les sécrétions mises en suspension dans une solution tamponnée aqueuse ou organique selon le cas subissent un traitement protéolytique et une purification selon les exemples 1 et 3 et/ou un traitement selon l'exemple 6.

## H.- ANTIADHESINES ISSUES DE VIRUS

### Exemple 12

Les virus obtenus à partir de fluides ou de cellules infectés sont purifiés par ultracentrifugation en gradient de densité.

Les glycoprotéines porteuses des structures oligosaccharidiques sont extraites après traitement par des détergents membranaires par le butanol selon une méthode dérivée de L.A. HUNT (Glycosidase analysis of Large acidic -type glycopeptides from viral and cellular membrane glycoproteins, Biochem. J., 1983, 209, 659-667).

Les antiadhésines sont obtenues par digestion enzymatique et purifiés selon les exemples 1 et 3.

## I.- ANTIADHESINES ISSUES DE PHAGES

### Exemple 13

Les phages sont isolés à partir de différents lisiers, d'épandages de fermes... selon la méthode de SMITH et Huggins, 1982 (Successful treatment of experimental E. coli infections in Mice using phage : its general superiority over antibiotics, J. of General Bacteriology, 128, 307-318). Les oligosaccharides sont obtenus par protéolyse selon l'exemple 1.

## J. - ANTIADHESINES OBTENUES A PARTIR DE BACTERIES

### Exemple 14

Les bactéries sont cultivées généralement à 37°C pendant 24 heures sur un milieu approprié à leur développement (exemples E. coli K88 sur bouillon trypticase-soja, E. coli K99 sur milieu Minca, les lactobacilles sur milieu de Rogosa...).

Les extraits bactériens sont obtenus par désintégration aux ultrasons, concentrés et soumis à l'hydrolyse selon les exemples 1 et 3 ou à l'extraction des structures lipidiques selon l'exemple 6.

## K. - ANTIADHESINES DE CHAMPIGNONS, LEVURES

### Exemple 15

Les organismes sont isolés, à partir d'extraits industriels ou des milieux de culture utilisés pour leur multiplication par centrifugation. Les extraits sont soumis à la protéolyse selon les exemples 1, 2, 3 ou 6.

## L. - ANTIADHESINES ISSUES DES ALGUES, MOUSSES, LICHENS, FOUGERES.

### Exemple 16

Les végétaux sont broyés, les glycoprotéines solubilisées dans une solution tamponnée et soumises à l'hydrolyse selon l'exemple 1, les antiadhésines purifiées selon l'exemple 3 ou 6.

## M. - ANTIADHESINES EXTRAITES DES VEGETAUX SUPERIEURS

### Exemple 17

Les végétaux accessibles à l'état de broyat total sec ou humide et susceptibles d'être fractionnés en deux composantes protéique et lipidique, sont soumis aux procédés décrits dans les exemples 1, 3 ou 6, après mise en solution ou mise en suspension si nécessaire.

## N. - ANTIADHESINES ISOLEES DE TISSUS OU ORGANES

Exemple 18

Les glycolipides et les glycopeptides sont obtenus par broyage des organes totaux tels que les cerveaux, reins, foies, poumons... Les glycolipides sont extraits selon l'Exemple 6. Les glycopeptides sont obtenus par une hydrolyse selon les Exemples 1, 2. Ils peuvent être séparés des glycosaminoglycanes par précipitation de ces derniers par le chlorure de cétylpyridinium et sont alors traités selon l'Exemple 3.

## O. - ANTIADHESINES ISOLEES D'OEUFS

Exemple 19

Les glycoprotéines de l'oeuf (telles que l'ovomucoïde, l'avidine, l'ovalbumine), récupérées après précipitation sélective du lysozyme et séparations chromatographiques, sont soumises à une protéolyse selon l'exemple 1, les antiadhésines sont concentrées et purifiées selon l'exemple 3.

## MISE EN EVIDENCE DE L'ACTIVITE INHIBITRICE DE L'ADHERENCE BACTERIENNE

Pour mettre en évidence l'activité des antiadhésines conforme à la présente invention, on a réalisé :
- des expérimentations in vitro sur des cibles auxquelles s'attachent les microorganismes étudiés,
- des expérimentations in vivo sur des animaux infecté volontairement par des microorganismes pathogènes,
- des essais cliniques.
Dans le cas des expérimentations in vitro, on a procédé de la façon suivante :

1. Méthodologie du test d'activité du produit inhibant l'adhésion des microorganismes sur les cellules cibles.

Le test d'adhérence est réalisé par la mise en contact, grâce à une incubation, des microorganismes et des cibles libres ou immobilisées. Les paramètres de concentrations respectives, de pH, de force ionique, de nature d'ions, de température et de durée de l'essai étant définis, le test d'inhibition de l'adhérence est réalisé soit par préincubation des antiadhésines, conforme à la présente invention, avec les microorganismes, soit par introduction des antiadhésines dans le milieu contenant l'agent microbien et la structure cible libre ou immobilisée sur un support.
L'observation et/ou la quantification de l'adhésion et de l'anti-adhésion sont faites :
- de visu, si cela est possible, selon le système considéré,
- en microscopie optique, soit en lecture directe après (ou sans) coloration, soit en contraste de phase,
- par spectrophotométrie,
- par mesure de la radioactivité ou de la fluorescence des microorganismes attachés aux cibles.
Cette dernière méthode assure une meilleure quantification du phénomène.

2. Description d'un test général d'adhésion et d'antiadhésion basé sur la mesure de la radioactivité des microorganismes attachés aux cellules cibles.

Principe

Le microorganisme examiné est mis en culture et marqué radioactivement grâce à l'incorporation de précurseurs dans son matériel constitutif.
La concentration finale du microorganisme est ajustée selon les conditions du test. Les structures cibles cellules et/ou leur mucus sont préparées selon les différentes techniques d'obtention de cellules décrites dans la littérature scientifique. Le critère de qualité recherché est l'aptitude de l'adhésion des microorganismes étudiés.

Test

Les cellules et/ou leur mucus sont immobilisées sur un support approprié permettant la mise à leur contact des microorganismes radioactifs en présence ou en l'absence des antiadhésines. Après des lavages successifs le matériel biologique (cibles plus bactéries radioactives adhérentes) est décroché du support par un traitement approprié et la radioactivité du milieu est comptée par scintillation liquide.
Dans chaque expérimentation, les témoins expriment le 100 % d'adhésion. Les essais permettent de quantifier l'activité biologique des antiadhésines rapportée à l'unité de masse de celles-ci.

3. Application de ce test général au cas de l'adhésion de E. coli K99 à des cellules cibles et/ou à leur mucus.

- E. coli K99 est cultivé sur un milieu approprié contenant du glucose et des vitamines comme par exemple le milieu de Minca (Guinée P.A.M. Jansen W.H. Agterberg C.M., 1976), auquel est ajouté une espèce chimique radioactive (acide aminé, nucléoside purique ou pyrimidique, acétate de sodium, etc...) précurseur susceptible d'être incorporé dans le matériel constitutif du microorganisme.

Après 18 h de culture, à 37°C afin que la synthèse du pili K99 ait lieu, ou à 18°C dans le cas contraire, les bactéries sont récupérées , lavées et mises en suspension dans une solution tamponnée pH 7,2 habituellement utilisée (HEPES-Hanks, tampon phosphate salin, ...).

Le numération bactérienne est faite par la mesure de la densité optique (DO) entre 420 et 662 nm (la DO étant fonction linéaire du nombre de colonies, DO = f(C)), et la concentration est ajustée à $10^8$-$10^9$ bactéries/ml. Des contrôles sont effectués sur la présence de pili K99 par séroagglutination avec des anticorps anti K99 et la viabilité des cellules bactériennes par dilution d'une prise aliquote et culture sur un milieu approprié.

- Préparation des cellules cibles et/ou des mucus et techniques d'immobilisation (d'après C.D. LAUX et al, 1984).

Le mucus est préparé à partir de l'intestin grêle de l'animal sacrifié (souris, lapereau, porcelet, agneau, veau...) par grattage et récupéré dans une solution tamponnée à pH 7,2. Une centrifugation à 28 000 g pendant 15 mintues permet l'élimination totale des débris et matériels cellulaires. La concentration en protéines de chaque préparation est contrôlée et ajustée au minimum à 3,5 mg/ml.

Les cellules sont préparées à partir de l'intestin grêle de l'animal (souris âgées de 5 à 8 semaines, lapereau d'une semaine, porcelet, agneau, veau âgé de moins de 5 jours) par grattage et récupération dans une solution tampon née utilisée pour la conservation des cellules. Une centrifugation à 1000 g élimine les débris tissulaires, une filtration et une centrifugation à 2000 g éliminent les contaminants. Les cellules des villosités intestinales sont isolées par trypsinisation (trypsine 0,1%) ou dilacération (Potter n°18) et la concentration ajustée entre $10^6$-$10^8$ cellules/ml.

Les préparations de cellules ou de mucus (0,25 ml) sont introduites dans les puits de plaques pour culture de tissus, incubées une nuit à 4°C, lavées deux fois par une solution tamponnée pH 7,2 pour enlever les protéines ou les cellules non fixées.

Les cellules d'espèces animales (lignées cellulaires établies ou cultures primaires) sont cultivées dans un milieu de culture cellulaire en présence de 10% de sérum de veau foetal, de pénicilline et de streptomycine. Elles sont incubées pendant 24 à 48 h à 37°C, en atmosphère contrôlée, afin d'obtenir une couche monocellulaire confluente dans les puits de plaque de culture. Avant utilisation, les cellules sont lavées pour enlever toute trace de milieu de culture.

- Adhésion et inhibition de l'adhésion

Les bactéries (0,25 ml,soit 50 000 dpm minimum incorporés) et 50 µl de tampon sont introduits dans les puits et incubés à 37°C avec les cellules et/ou le mucus immobilisés.

- Après lavages, les bactéries adhérentes sont décrochées par l'action de 0,5 ml de SDS 0,5% ou de tout autre agent détersif ou chélateur, pendant 2 heures à 37°C.

Les échantillons prélevés (0,25 ml) sont ensuite analysés par comptage de la radioactivité en scintillation liquide.

Dans le test d'inhibition de l'adhérence,le médicament conforme à l'invention est introduit au milieu ci-dessus décrit dans 50 µl du tampon accompagnant les bactéries.

- Expression des résultats

Les résultats sont exprimés en quantité de radioactivité récupérée dans les puits, dans les conditions définies ci-dessus et comparativement aux témoins avec lesquels est établi le 100% d'adhérence.

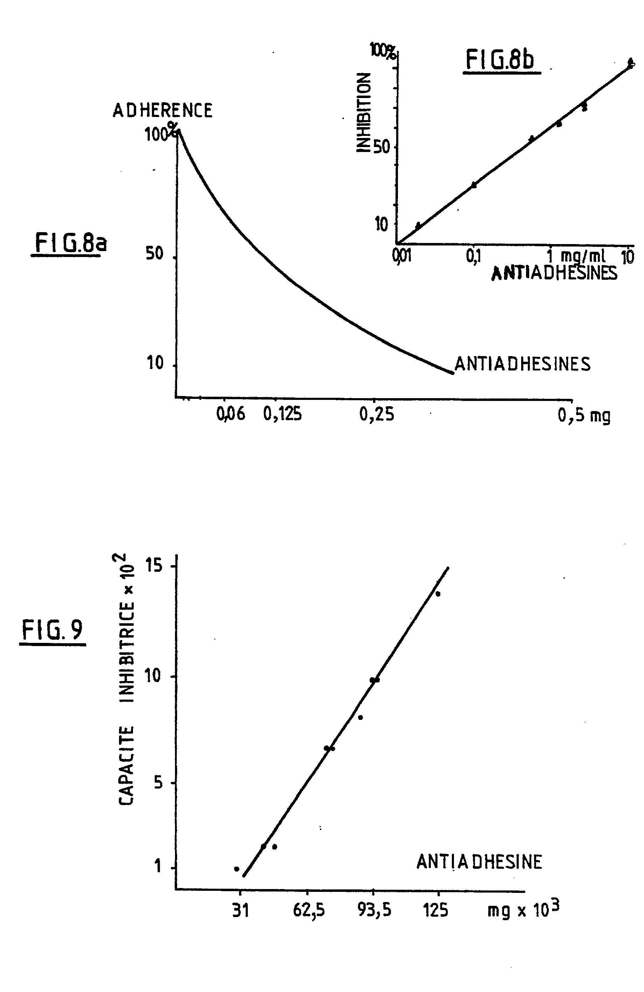

Une courbe type des résultats habituellement obtenus est représentée sur les figures 8a et 8b à titre d'exemple et sans que cela soit limitatif. L'inhibition est une fonction linéaire du logarithme de la concentration en médicament. Les sucres simples de masse moléculaire de l'ordre de 200, ou les oligosaccharides de masse moléculaire de l'ordre de 400,donc de masse inférieure à 1000, n'ont aucune action inhibitrice ou très faiblement inhibitrice.

4. Test d'inhibition de l'hémagglutination résistante au mannose.

Autre modèle d'étude de l'adhésion des microorganismes sur une cellule cible.

a) Principe

Les bactéries ($10^7$-$10^9$ bactéries/ml) en concentration égale à 10 unités d'hémagglutination,( une unité correspondant à la plus grande dilution de la suspension bactérienne capable d'agglutiner les globules rouges de l'espèce choisie en présence de mannose) sont mises en suspension dans 24 µl de solution isotonique tamponnée (pH 6,8 - 7,2) et incubées en présence de 50 µl ou en l'absence de médicaments conforme à la présente invention pendant 10 minutes à 4°C ou à température ambiante. Les érythrocytes à 3 % (v/v) sont ensuite ajoutés dans 25 µl au milieu d'incubation. La lecture (oeil, microscope ou turbidimètre ou spectrophotomètre) est fait après 30 minutes de contact.

b) Applications aux colibacilles

Les systèmes utilisés sont décrits dans le Tableau I.

### TABLEAU I

| E. coli pathogène pour | pili principal | origine des globules rouges |
|---|---|---|
| Veau, agneau, porcelet | K99 | homme, mouton, cheval |
| porcelet | K88 | cobaye |
| Homme | CFA I/II | bovin, poulet |

C) Application à E.coli K99

La capacité inhibitrice du médicament, à savoir l'inverse de la concentration minimale capable d'inhiber l'agglutination des globules rouges de mouton par les E.coli K99, est proportionnelle à la quantité des substances conformes à la présente invention (cf. fig.9).

5. Test d'adhérence et d'activité des antiadhésines réalisé sur des cellules ou des villosités par observation microscopique. Application à l'adhérence d'E. coli K99 sur les entérocytes de veau.

Les villosités ou les cellules obtenues selon la technique décrite ci-dessus sont utilisées soit immédiatement, soit après congélation à -20°C dans un milieu de HANKS ou tout milieu adapté à la conservation des cellules. Dans le cas d'utilisation de préparations congelées, les additifs sont éliminés par lavages successifs.

Les bactéries ($10^7$-$10^{11}$ /ml) sont mises en contact avec la préparation cellulaire ($10^7$ cellules/ml) pendant 40 minutes à 20°C ou 37°C selon les systèmes dans un intervalle de pH allant de 5,5 à 8,5 , en présence ou en l'absence du (ou des) médicament(s) conforme(s) à l'invention.

L'adhérence est positive lorsque plus de 15 bactéries sont fixées à la surface de la cellule.

L'inhibition s'exprime par la formule :

$$\frac{\text{nombre de cellules sans bactéries}}{\text{nombre de cellules avec bactéries + cellules sans bactéries}} \times 100$$

Avec $2 \times 10^5$ cellules (200µl) mises en contact avec $2 \times 10^7$ bactéries (200 µl) pendant 40 minutes à 37°C en présence de 0,25 mg de produit conforme à la présente invention, l'inhibition observée est de 60-65 % comparativement au témoin.

Dans le cas des expérimentations in vivo, on procède de la façon suivante :

1) Inoculation expérimentale de souriceaux par E. coli K99

Des lots de souriceaux de même race (SWISS, OFI, CD...) âgés de moins de 48 heures sont infectés oralement par $5.10^4$, $10^5$ E. coli K99. L'évolution de la population est suivie pendant 3 jours (J) après une seule administration orale du médicament conforme à la présente invention, dans les 30 minutes qui suivent l'infection ou en l'absence de traitement.

Dans le cas d'une infection DL 100 dans les 3 jours qui suivent l'inoculation ($5.10^4$-$10^5$ E.coli K99/individu) la protection de la population est montrée dans le Tableau II suivant :

## TABLEAU II

### Evolution des populations exprimée en % de survivants

| Expériences | Inoculum | | | | | |
|---|---|---|---|---|---|---|
| | $5.10^4$ | | $10.^5$ | | | |
| Médicament mg/kg | Témoins 0 | Traités 0,6 | Témoins 0 | 0,6 | Traités 7,2 | 12 |
| J 1 | 100 | 100 | 40 | 68 | 83 | 100 |
| J 2 | 20 | 40 | 0 | 12 | 66 | 67 |
| J 3 | 0 | 20 | 0 | 0 | 39 | 39 |
| Echantil-lonage | 12 | 14 | 24 | 14 | 18 | 18 |

2) Inoculation expérimentale de veaux nouveaux-nés par E.coli K99

La diarrhée est reproduite selon la technique dérivée de celle décrite par SMITH H.W. et HALL, S.S. (1967). Le veau, âgé de moins de 8 heures, dépourvu de colostrum, est infecté oralement par une dose mortelle de 10$^{10}$ E. coli K99. Le lot témoin, non traité, meurt en moins de 48 heures après déshydratation. Les veaux essais sont traités par administration orale des antiadhésines conforme à la présente invention.

Plusieurs modes de traitement se sont révélés efficaces, après administration dans les deux heures qui suivent l'inoculation ou dès le déclenchement de la diarrhée. Tous les animaux traités ont guéri.

L'amélioration clinique a été rapide et la guérison observée en moins de 48 heures. L'efficacité du produit se traduit par un retour à la normale des paramètres biochimiques tels l'ionogramme sérique ou d'excrétion, le pH des fécès, un arrêt de la fuite hydrique. Des veaux traités et gardés en observation pendant 90 jours, n'ont plus présenté de troubles de gastroentérites et ont poursuivi une croissance normale.

L'activité d'antiadhésine est prouvée par l'étude de la flore adhérente à la paroi intestinale.

Des numérations bactériennes au niveau du duodénum, de jéjunum et de l'iléon ont été effectuées après grattage de la paroi intestinale d'animaux témoins et d'animaux traités. Pour ce faire, et afin de distinguer la flore pathogène d'une flore intestinal colibacillaire adhérente éventuellement présente, l'inoculum possède un caractère d'antibio-résistance. Ainsi la résistance à l'acide nalidixique (Na$\ell$r) constitue le marqueur de la souche E. coli K99.

Chez les veaux traités, la flore pathogène E.coli K99 Na$\ell$r adhérente diminue d'au-moins deux logarithmes par rapport à la flore des veaux témoins non traités. Les résultats obtenus sont présentés dans le Tableau III.

## TABLEAU III

Flore pathogène Naℓr adhérente sur la paroi intestinale, en log

| | Duodénum | | | Jéjunum | | | Iléon | | |
|---|---|---|---|---|---|---|---|---|---|
| | Moyen- ne | Ecart type | Va- riance | Moyen- ne | Ecart type | Va- riance | Moyen- ne | Ecart type | Va- riance |
| Essais (7) | 4,36 | 1,10 | 1,21 | 5,20 | 1,58 | 2,50 | 5,61 | 1,08 | 0,97 |
| Témoins (4) | 5,95 | 0,64 | 0,41 | 8,07 | 0,92 | 0,84 | 8,15 | 1,17 | 0,95 |

$t = 2,61$     $t = 3,28$     $t = 3,87$

différence significative     différence hautement significative     hautement significative

$t : 0,05 = 2,26$

$t : 0,01 = 3,25$

### 3) Essais cliniques

Dans la présente invention, des essais de terrain ont permis d'établir un diagnostic étiologique de la diarrhée infectieuse d'origine colibacillaire, virale ou parasitaire par un contrôle systématique effectué à partir des selles prélevées avant la thérapeutique.

L'efficacité du médicament est mise en regard du diagnostic. Les veaux diarrhéiques âgés de moins de 5 jours sujets aux attaques colibacillaires K99 positive et les veaux plus âgés sont guéris par les antiadhésines à un fort pourcentage comme l'indiquent les résultats des Tableaux IV et V, résultats obtenus sur deux campagnes et sur 219 veaux.

L'efficacité est d'autant plus grande que l'administration des andtiadhésines est précoce et menée dès les premiers symptômes du trouble d'entérogastrite. Il est à noter que l'alimentation maternelle n'a pas été supprimée.

Les antiadhésines sont efficaces en curatif dans les conditions normales d'utilisation. Un à deux jours d'administration du médicament (1 à 10 x 60 mg) a été la durée nécessaire pour traiter efficacement les diarrhées d'origine colibacillaire, virale ou parasitaire.

## TABLEAU IV

## Veaux âgés de moins de 5 jours.

| Résultats des analyses de fécès | E. coli K99+ seuls ou en association | | | Rota-virus | E.coli K99- seuls ou en association | | Analyses néga-tives | Analyses incom-plètes | TOTAL |
|---|---|---|---|---|---|---|---|---|---|
| | K99+ | K99+ rotavirus | K99+ rotavirus K99- | Rotavirus | K99- pathogènes | K99- et coccidies | Pas d'agents infectieux | Pas de recherches bactério-logiques ou virologiques | |
| Veaux traités | 15 | 8 | 8 | 25 | 18 | 11 | 11 | 3 | 99 |
| Veaux guéris | 13 | 6 | 4 | 18 | 18 | 8 | 11 | - | |
| Nombre de doses administrées/ animal | 3 | 2 | 2 | 7 | 10 | 2 | 10 | 10 | 594 |
| Nombre d'animaux guéris | 23 | | | 18 | 26 | | 11 | | 78 |
| % d'animaux guéris avec 6 doses en moyenne | | | | | | | | | 78 |

4) Innocuité du médicament

En laboratoire, aucune mortalité n'a été enregistrée lors de l'ingestion du médicament conforme à la présente invention tant sur des souriceaux que sur des veaux recevant quotidiennement 120 mg et ce pendant 90 jours. La croissance des animaux n'est pas altérée. Aucun effet secondaire n'a été observé pendant l'expérimentation. Ce médicament d'origine biologique, de la classe des antiadhérents, présente une innocuité totale, tant par os que par voie intrapéritonéale.

EP 0 201 390 B1

## TABLEAU V

Veaux diarrhéiques de plus de 5 jours traités et guéris par les Antiadhésines de la présente invention (après 5 jours, la flore E. coli K99$^+$ n'est plus dominante)

| Résultats des analyses de fécès | VIRUS | | | | | | BACTERIES | | | | DIVERS | | TOTAL |
|---|---|---|---|---|---|---|---|---|---|---|---|---|---|
| | | | | Rotavirus associés avec | | | Agents microbiens | | | | | | |
| | Rotavirus | Coronavirus K99- | Rota et Coronavirus | K99- strepto fecalis ou Faecum | cryptosporidie | E. coli K99- entéropathogène | K99 entéropathogène | K99- strepto fecalis ou faecum | K99- et autres agents | strepto no proteus | Recherches négatives | Recherches incomplètes | |
| Veaux traités | 7 | 1 | 8 | 11 | 4 | 46 | 27 | 4 | 4 | 2 | 2 | 4 | 120 |
| Veaux guéris | 6 | - | 5 | 10 | 3 | 40 | 26 | 4 | 4 | 2 | 2 | 4 | 106 |
| Nombre moyen de doses administrées/animal | 6 | 8 | 7 | 4 | 5 | 7 | 6 | 4 | 4 | 3 | 5 | 4 | 718 |
| Nombre d'animaux guéris | 64 | | | | | | 42 | | | | 6 | | |
| % d'animaux guéris avec 6 doses en moyenne | | | | | | | | | | | | | 88 |

L'expérimentation a porté sur 219 veaux diarrhéiques. Seuls sont enregistrés comme guéris les animaux ayant reçu les antiadhésines décrites dans l'invention à l'exception de tous autres traitements ou - compléments jugés utiles par le vétérinaire ou l'éleveur. Posologie du traitement : les antiadhésines ont été administrées sur demande de l'éleveur, souvent après constatation d'une diarrhée profuse ce que explique une administration prolongée chez certains animaux.

**Revendications**

1. Procédé de préparation d'inhibiteurs de l'adhésion cellulaire ou antiadhésines, lequel procédé est caractérisé en ce que l'on lyse enzymatiquement et/ou chimiquement les glycoprotéines et/ou les glycopeptides, les structures lipidiques et/ou les glycolipides contenus ou provenant de différents substrats organiques liquides ou solides, en ce que l'on recueille les structures resistantes à la lyse, de nature partiellement ou totalement osidique, de masse supérieure à 1000 et en ce que l'on procède à leur stérilisation.

2. Procédé selon la revendication 1, caractérisé en ce que les glycoprotéines et/ou les glycolipides sont traités sans les avoir extraits du substrat organique.

3. Procédé selon la revendication 1, caractérisé en ce que l'on procède à la lyse des glycoprotéines et/ou des glycolipides après leur extraction du milieu biologique les contenant.

4. Procédé selon les revendications 1 et 3, caractérisé en ce que les glycoprotéines plasmatiques sont extraites par:
a) traitement en milieu organique par de l'éthanol en présence de caprylate de sodium suivi de
b) traitement thermique (60 à 70°C) et séparation des glycoprotéines précipitées et
c) soumission à l'action de protéolyse et traitement chimique supplémentaire permettant d'obtenir les structures antiadhésines de masse supérieure à 1000.

5. Procédé selon les revendications 1 et 3, caractérisé en ce que les glycolipides sont extraits par action d'un mélange de solvants organiques, puis soumis à une lyse chimique permettant d'obtenir les antiadhésines.

6. Procédé selon la revendication 1, caractérisé en ce que la lyse est effectuée:
1) dans le cas des glycoprotéines, à l'aide d'une protéase et de manière préférentielle à l'aide de la thermolysine en présence d'ions calcium à un pH compris entre 5 et 7 et à une température inférieure ou égale à 80°C suivie d'une hydrazinolyse ou traitement chimique analogue permettant de libérer les antiadhésines constitutives,
2) dans le cas des glycolipides par une hydrolyse alcaline douce souvie d'une acétolyse ou traitement analogue permettant de libérer les antiadhésines constitutives.

7. Procédé selon l'une quelconque des revendications 1 à 6, caractérisé en ce que l'on procède à la purification des antiadhésines par l'une des quelconques méthodes séparatives puis à leur concentration et stérilisation.

8. Utilisation de substances de type oligosaccharidiques, de masse moléculaire supérieure à 1000, et comprenant au minimum 3 oses simples ou substitués, l'un des oses étant obligatoirement le glucose ou le galactose, obtenues selon l'une quelconque des revendications 1 à 7, pour l'obtention de produits inhibiteurs de l'adhésion cellulaire.

9. Utilisation suivant la revendication 8, dans laquelle lesdites substances de nature oligosaccharidiques sont greffées sur un support protéique ou lipidique.

10. Utilisation suivant l'une quelconque des revendications 8 ou 9, caractérisée en ce que lesdites substances de nature oligosaccharidiques sont incorporées dans un médicament.

11. Utilisation suivant l'une quelconque des revendications 8 ou 9, caractérisée en ce que lesdites substances de nature oligosaccharidiques sont incorporées dans un aliment ou un additif pour aliment.

**Claims**

1. A process for the preparation of cell adhesion inhibitors, or antiadhesins, which process is characterized in that the glycoproteins and/or the glycopeptides, the lipid structures and/or the glycolipids contained in or originating from different liquid or solid organic substrates are enzymatically and/or chemically lyzed and in that the structures resistant to lysis, which are of partially or totally osidic character and whose molecular weight is always greater than 1000, are collected and sterilized.

2. A process according to Claim 1, characterized in that the glycoproteins and/or the glycolipids are treated without being extracted from the organic substrate.

3. A process according to Claim 1, characterized in that the glycoproteins and/or the glycolipids are lyzed after they have been extracted from the biological medium in which they are present.

4. A process according to Claims 1 and 3, characterized in that the plasma glycoproteins are extracted by:
a) treatment in an organic medium with ethanol in the presence of sodium caprylate, followed by
b) heat treatment (60 to 70°C) and separation of the precipitated glycoproteins, and
c) the action of proteolysis and additional chemical treatment making it possible to obtain the antiadhesin structures with a molecular weight greater than 1000.

5. A process according to Claims 1 and 3, characterized in that the glycolipids are extracted by the action of a mixture of organic solvents, and then subjected to chemical lysis making it possible to obtain the antiadhesins.

6. A process according to Claim 1, characterized in that the lysis is carried out:

1) in the case of glycoproteins, with the aid of a protease and, preferably, with the aid of thermolysin in the presence of calcium ions at a pH of between 5 and 7 and at a temperature below or equal to 80°C, followed by hydrazinolysis or an analogous chemical treatment making it possible to free the constituent antiadhesins;

2) in the case of glycolipids, by mild alkaline hydrolysis followed by acetolysis or an analogous treatment making it possible to free the constituent antiadhesins.

7. A process according to any one of Claims 1 to 6, characterized in that the antiadhesins are purified by any one of the methods of separation and then concentrated and sterilized.

8. Use of oligosaccharide-type substances with a molecular weight greater than 1000 and comprising at least 3 simple or substituted oses, one of the oses necessarily being glucose or galactose, said substances being obtained according to any one of Claims 1 to 7, for the preparation of products for inhibiting cell adhesion.

9. Use according to Claim 8 in which said substances of oligosaccharide character are grafted onto a protein or lipid support.

10. Use according to Claim 8 or Claim 9, characterized in that said substances of oligosaccharide character are incorporated into a drug.

11. Use according to Claim 8 or Claim 9, characterized in that said substances of oligosaccharide character are incorporated into a food or a food additive.

## Patentansprüche

1. Verfahren zur Herstellung von Zelladhäsionsinhibitoren oder Anti-Adhäsinen, dadurch gekennzeichnet, daß man enzymatisch und/oder chemisch Glycoproteine und/oder Glycopeptide, Lipidstrukturen und/oder Glycolipide, die in verschiedenen flüssigen oder festen organischen Substraten enthalten sind oder aus ihnen stammen, lysiert, daß man die teilweise oder völlig Lyse-resistenten Strukturen mit einem Molekulargewicht größer als 1000 gewinnt und daß man sie sterilisiert.

2. Verfahren nach Anspruch 1, dadurch gekennzeichnet, daß die Glycoproteine und/oder Glycolipide ohne Extraktion aus dem organischen Substrat behandelt werden.

3. Verfahren nach Anspruch 1, dadurch gekennzeichnet, daß man die Glycoproteine und/oder die Glycolipide nach Extraktion aus dem sie enthaltenden biologischen Milieu lysiert.

4. Verfahren nach den Ansprüchen 1 und 3, dadurch gekennzeichnet, daß man die Plasma-Glycoproteine durch

a) Behandlung in organischem Milieu mit Ethanol in Gegenwart von Natriumcaprylat, gefolgt von

b) thermischer Behandlung (60 bis 70°C) und Abtrennen der präzipitierten Glycoproteine und

c) Unterwerfen einer Proteolyse und ergänzenden chemischen Behandlung, wodurch die Anti-Adhäsinstrukturen mit einem Molekulargewicht von größer als 1000 erhalten werden, extrahiert.

5. Verfahren nach den Ansprüchen 1 und 3, dadurch gekennzeichnet, daß man die Glycolipide mit einem Gemisch aus organischen Lösungsmitteln extrahiert und sie einer chemischen Lyse unterwirft, wobei die Anti-Adhäsine erhalten werden.

6. Verfahren nach Anspruch 1, dadurch gekennzeichnet, daß die Lyse

1) im Falle der Glycoproteine mit Hilfe einer Protease, bevorzugt Thermolysin, in Gegenwart von Calciumionen bei einem pH zwischen 5 und 7 und einer Temperatur von kleiner oder gleich 80°C, gefolgt von einer Hydrazinolyse oder analogen chemischen Behandlung, wodurch die grundlegenden Anti-Adhäsine freigesetzt werden,

2) im Falle von Glycolipiden durch eine milde alkalische Hydrolyse, gefolgt von einer Acetolyse oder einer analogen Behandlung, wodurch die grundlegenden Anti-Adhäsine freigesetzt werden, durchgeführt wird.

7. Verfahren nach einem der Ansprüche 1 bis 6, dadurch gekennzeichnet, daß man die Anti-Adhäsine nach irgendeinem Trennverfahren reinigt, anschließend sie konzentriert und sterilisiert.

8. Verwendung von Substanzen des Oligosaccharid-Typs mit einem Molekulargewicht größer als 1000 und mit mindestens 3 einfachen oder substituierten Einheiten, wovon eine obligatorisch Glucose oder Galactose ist, die nach einem der Ansprüche 1 bis 7 erhalten wurden, als Zelladhäsionsinhibitoren.

9. Verwendung nach Anspruch 8, dadurch gekennzeichnet, daß die Substanzen vom Oligosaccharid-Typ auf einem proteinartigen oder lipidartigen Träger befestigt sind.

10. Verwendung nach einem der Ansprüche 8 oder 9, dadurch gekennzeichnet, daß die Substanzen vom Oligosaccharid-Typ in ein Medikament eingearbeitet sind.

11. Verwendung nach einem der Ansprüche 8 oder 9, dadurch gekennzeichnet, daß die Substanzen vom Oligosaccharid-Typ in ein Lebensmittel oder einen Lebensmittelzusatzstoff eingearbeitet sind.

EP 0 201 390 B1

$$(Man)_{o\ \grave{a}\ n} \xrightarrow[\alpha 1 \rightarrow 2]{} Man$$

$$\alpha 1 \rightarrow 3$$

$$\beta 1 \rightarrow 6$$

$$Man \xrightarrow[\beta 1 \rightarrow 4]{} glc\ Nac \xrightarrow[\beta 1 \rightarrow 4]{} glc\ Nac$$

$$(Man)_{o\ \grave{a}\ n} \xrightarrow[\substack{\alpha 1 \rightarrow 3\ et/ \\ ou\ 6}]{} Man$$

FIG. 1

Fuc

$$\alpha 1 \rightarrow 6$$

Xyl

$$\beta 1 \rightarrow 6$$

$$Man_{(o\ \grave{a}\ n)} \xrightarrow[\alpha 1 \rightarrow 2]{} Man \xrightarrow[\alpha 1 \rightarrow 3]{} glc\ Nac \xrightarrow[\beta 1 \rightarrow 4]{} glc\ Nac\ -$$

FIG. 2

EP 0 201 390 B1

# FIG. 3

AS ( 0 à n).

↓ $\alpha 2 \rightarrow 3$ ou 6

gal ( 1 ou 0)

↓ $\beta 1 \rightarrow 4$

glc Nac ( 1 ou 0)

↓ $\beta 1 \rightarrow 4$

AS(0 à n) — AS(0 à n) → gal ( 0 à 4) → glc Nac(0 ou 1) ————→ Man
$\alpha 2 \rightarrow 8$   ou   $\alpha 2 \rightarrow 3$ ou 6      $\beta 1 \rightarrow 4$ ou glc        $\beta 1 \rightarrow 2$.     $\alpha 1 \rightarrow 3$

Fuc                (gal — glc Nac) (1 à 4) ————→ Man ——→ glc Nac —→ glc Nac —→
$\beta 1 \rightarrow 4$            $\beta 1 \rightarrow 4$      $\beta 1 \rightarrow 4$   $\beta \rightarrow 1$

$\alpha 1 \rightarrow 6$        ( Fu )0 à 1

AS ( 0 à n) ————→ gal ( 0 à n) ————→ glc Nac ( 0 à 1) ————→ Man
$\alpha 2 \rightarrow 3$ ou 6              $\beta 1 \rightarrow 4$                $\beta 1 \rightarrow 2$      ↑ $\beta 1$   6

ou                                                              glc Nac ( 1 ou 0)

Fuc

gal ( 1 ou 0 )

↑ $\alpha 2 \rightarrow 3$ ou 6

AS ( 0 à n)

FIG. 4

## FIG.5

## FIG.6

## FIG.7

FIG.8a

FIG.8b

FIG.9